# EUROPEAN PATENT APPLICATION

(11) **EP 4 729 533 A1**
(43) Date of publication of application: **22.04.2026**
(21) Application number: 24823746.3
(22) Date of filing: 14.06.2024
(51) Int. Cl.: C07K 14/775, C07K 14/705, C07K 16/28, A61P 35/00, A61K 38/00, A61K 39/00

(54) **FUSION PROTEIN COMPRISING APOLIPOPROTEIN OR FRAGMENT THEREOF, AND USE THEREOF**

(30) Priority: 15.06.2023 KR 20230077013; 13.06.2024 KR 20240077168
(71) Applicant: RNAGENE Inc., Seoul 05855 (KR)
(72) Inventor: LEE, Woo Ghil, Seoul 05834 (KR)
(74) Representative: Murgitroyd & Company
(86) International application number: PCT/KR2024/008224
(87) International publication number: WO 2024/258229

(57) **Abstract**

The present disclosure relates to a fusion protein and uses thereof, the fusion protein including an apolipoprotein or a fragment thereof and a targeting protein or a fragment thereof. According to an aspect of the fusion protein, it has been confirmed that a lipid-based carrier including the fusion protein exhibits significantly improved targeting efficiency, thereby enabling effective delivery of an active substance to a desired target.

## Description

### Technical Field

The present disclosure relates to a fusion protein and uses thereof, the fusion protein including apolipoprotein or a fragment thereof and a targeting protein or a fragment thereof.

### Background Art

As is well known, the development of a single drug requires a substantial amount of cost and time. Accordingly, various approaches have been studied to improve the safety and efficacy of existing drugs, one of which is the research field of drug delivery systems (DDS). The DDS aims to efficiently deliver drugs to a desired target, thereby reducing drug-induced side effects while maximizing drug efficacy.

Drug delivery may be largely divided into two concepts. The first concept is that of a prodrug. A prodrug itself is pharmacologically inactive outside the body, but exhibits therapeutic effects once it is metabolized by metabolic enzymes inside the body. In other words, this approach involves inducing the desired pharmacological activity at the target site through chemical modification of the drug structure. The second concept is the direct delivery of a drug to a target molecule, based on a drug delivery system. This concept is further divided into drug delivery systems utilizing polymers and drug delivery device systems based on fusion technologies.

Drug delivery systems may be classified according to the route of administration, the type of delivery technology, and the type of drug. Based on the route of administration, they are generally categorized into oral, injectable, pulmonary inhalation, transdermal, implantable, and other types. Based on the type of delivery technology, they may be classified into absorption-enhancing, sustained-release, target-site-focused, and intelligent DDS types. In the drug delivery systems described above, microparticles or microspheres may be used as carriers for drug delivery. It is important to design the formulation of these drug carriers so that so that therapeutic agents may be efficiently delivered to the treatment site, thereby reducing drug side effects, improving patient compliance with the drugs, and maximizing drug efficacy and effectiveness.

Accordingly, the present inventors have developed a carrier capable of effectively delivering a drug to a target site, thereby completing the present disclosure.

### Disclosure

### Technical Problem

An aspect provides a fusion protein including 1) an apolipoprotein or a fragment thereof, and 2) a targeting protein or a fragment thereof.

Another aspect provides a polynucleotide including a sequence encoding the fusion protein.

Another aspect provides an expression vector including the polynucleotide.

Another aspect provides a targeting carrier including the fusion protein and a lipid-based carrier.

Another aspect provides a composition for drug delivery including the targeting carrier and a drug of interest.

Another aspect provides a pharmaceutical composition including the targeting carrier and a drug of interest.

### Technical Solution

An aspect provides a fusion protein including 1) an apolipoprotein or a fragment thereof; and 2) a targeting protein or a fragment thereof.

The apolipoprotein may include one or more selected from the group consisting of apolipoprotein A (ApoA protein), apolipoprotein B (ApoB protein), apolipoprotein C (ApoC protein), apolipoprotein D (ApoD protein), apolipoprotein E (ApoE protein), apolipoprotein F (ApoF protein), apolipoprotein H (ApoH protein), apolipoprotein L (ApoL protein), apolipoprotein M (ApoM protein), and apolipoprotein(a) (Apo(a) protein).

The apolipoprotein or a fragment thereof may include an ApoE-derived peptide.

The ApoA protein may include one or more selected from the group consisting of Apo-AI, Apo-A2, Apo-A4, and Apo-A5.

The ApoB protein may include one or more selected from the group consisting of Apo-B48 and Apo B-100.

The ApoC protein may include one or more selected from the group consisting of ApoC-I, ApoC-II, ApoC-III, and ApoC-IV.

The ApoE protein may include one or more selected from the group consisting of ApoE1, ApoE2, ApoE3, ApoE4, ApoE5, ApoE6, ApoE7, ApoE8, ApoE9, ApoE10 and ApoE11, and may be specifically ApoE3 or ApoE4.

The apolipoprotein or a fragment thereof may include a lipid-binding region of the apolipoprotein. The lipid-binding region may refer to the 244th to 272nd amino acids from the N-terminus of an apolipoprotein protein, specifically, the 244th to 272nd amino acids from the N-terminus of an ApoE protein, and more specifically, may be a peptide represented by the 244th to 272nd amino acid sequence of SEQ ID NO: 1 or SEQ ID NO: 2.

The fragment of the apolipoprotein may be one in which a receptor-binding region is deleted. The receptor-binding region may refer to the 136th to 150th amino acids from the N-terminus of an apolipoprotein, specifically, the 136th to 150th amino acids from the N-terminus of an ApoE protein, and more specifically, may be a peptide represented by the 136th to 150th amino acid sequence of SEQ ID NO: 1 or SEQ ID NO: 2.

The fragment of the apolipoprotein may be one in which both a receptor-binding region and a hinge region are deleted. The hinge region may refer to the 168th to 205th amino acids from the N-terminus of the apolipoprotein, specifically, the 168th to 205th amino acids from the N-terminus of the ApoE protein, and more specifically, may be a peptide expressed by the 168th to 205th amino acid sequence of SEQ ID NO: 1 or SEQ ID NO: 2.

The fragment of the apolipoprotein may be a partially deleted form of the apolipoprotein in which 100 to 150 amino acids are sequentially deleted from the N-terminus of the apolipoprotein. Specifically, the fragment may include a polypeptide represented by an amino acid sequence in which 100 to 150, 110 to 150, 120 to 150, 130 to 150, 140 to 150, or 145 to 150 amino acids are deleted from the N-terminus.

The fragment of the apolipoprotein may also be a partially deleted form of the apolipoprotein in which 151 to 220 amino acids are sequentially deleted from the N-terminus of the apolipoprotein, specifically, the fragment may include a polypeptide represented by an amino acid sequence in which 151 to 220, 160 to 220, 170 to 220, 180 to 220, 190 to 220, 195 to 220, 200 to 220, 205 to 220, 210 to 220, 215 to 220, 151 to 215, 160 to 215, 170 to 215, 180 to 215, 190 to 215, 195 to 215, 200 to 215, 205 to 215, 151 to 210, 160 to 210, 170 to 210, 180 to 210, 190 to 210, 195 to 210, 200 to 210, 205 to 210, 151 to 205, 160 to 205, 170 to 205, 180 to 205, 190 to 205, 195 to 205, or 200 to 205 amino acids are deleted from the N-terminus.

In an embodiment, the apolipoprotein fragment may include polypeptide represented by an amino acid sequence in which 150 amino acids are sequentially deleted from the N-terminus of the ApoE protein, or a polypeptide represented by an amino acid sequence in which 205 amino acids are deleted.

In an embodiment, the apolipoprotein may include a polypeptide represented by the amino acid sequence of SEQ ID NO: 1, and specifically may consist of a polypeptide represented by the amino acid sequence of SEQ ID NO: 1. In addition, any amino acid sequence that exhibits a homology of 80% or more, specifically 90% or more, more specifically 95% or more, even more specifically 98% or more, and most specifically 99% or more to the above sequence, and that substantially exhibits the same or corresponding activity as the apolipoprotein, is included without limitation.

In an embodiment, the apolipoprotein may include a polypeptide represented by the amino acid sequence of SEQ ID NO: 2, and may specifically consist of a polypeptide represented by the amino acid sequence of SEQ ID NO: 2. In addition, any amino acid sequence that exhibits a homology of 80% or more, specifically 90% or more, more specifically 95% or more, even more specifically 98% or more, and most specifically 99% or more to the above sequence, and that substantially exhibits the same or corresponding activity as the apolipoprotein, is included without limitation.

In an embodiment, the fragment of the apolipoprotein may include a polypeptide represented by an amino acid sequence in which 100 to 220 amino acids are deleted from the N-terminus of SEQ ID NO: 1 or SEQ ID NO: 2, specifically, it may include a polypeptide represented by an amino acid sequence in which 100 to 150, 110 to 150, 120 to 150, 130 to 150, 140 to 150, 145 to 150, 151 to 220, 160 to 220, 170 to 220, 180 to 220, 190 to 220, 195 to 220, 200 to 220, 205 to 220, 210 to 220, 215 to 220, 151 to 215, 160 to 215, 170 to 215, 180 to 215, 190 to 215, 195 to 215, 200 to 215, 205 to 215, 151 to 210, 160 to 210, 170 to 210, 180 to 210, 190 to 210, 195 to 210, 200 to 210, 205 to 210, 151 to 205, 160 to 205, 170 to 205, 180 to 205, 190 to 205, 195 to 205, or 200 to 205 amino acids are deleted, and more specifically, the fragment may include a polypeptide represented by an amino acid sequence in which 150 amino acids are sequentially deleted sequentially from the N-terminus of the amino acid sequence of SEQ ID NO: 2, or a polypeptide represented by an amino acid sequence in which 205 amino acids are sequentially deleted sequentially from the N-terminus.

In an embodiment, the fragment of the apolipoprotein may include a polypeptide represented by the amino acid sequence of SEQ ID NO: 3, and may specifically consist of a polypeptide represented by the amino acid sequence of SEQ ID NO: 3. In addition, any amino acid sequence that exhibits a homology of 80% or more, specifically 90% or more, more specifically 95% or more, even more specifically 98% or more, and most specifically 99% or more to the above sequence, and that substantially exhibits the same or corresponding activity as a fragment of the apolipoprotein, is included without limitation.

In an embodiment, the fragment of the apolipoprotein may include a polypeptide represented by the amino acid sequence of SEQ ID NO: 4, and may specifically consist of a polypeptide represented by the amino acid sequence of SEQ ID NO: 4. In addition, any amino acid sequence that exhibits a homology of 80% or more, specifically 90% or more, more specifically 95% or more, even more specifically 98% or more, and most specifically 99% or more to the above sequence, and that substantially exhibits the same or corresponding activity as a fragment of the apolipoprotein, is included without limitation.

As used herein, the term "homology" refers to the degree of similarity between nucleotide sequences encoding a protein or between amino acid sequences, and when the degree of homology is sufficiently high, the expression product of the corresponding gene may have the same or similar activity. Additionally, the degree of homology may be represented as a percentage based on the extent of sequence identity to a given amino acid or nucleotide sequence. As used herein, a sequence exhibiting the same or similar activity as a given amino acid or nucleotide sequence is represented as having a certain "% homology." For example, it may be determined by comparing sequences using standard software that calculates parameters such as score, identity, and similarity - specifically, using BLAST 2.0 - or by performing hybridization experiments under defined stringent conditions. The appropriate hybridization conditions fall within the scope of the art and may be determined by well-known methods (for example, J. Sambrook et al., Molecular Cloning: A Laboratory Manual, 2nd Edition, Cold Spring Harbor Laboratory Press, Cold Spring Harbor, New York, 1989; F.M. Ausubel et al., Current Protocols in Molecular Biology, John Wiley & Sons, Inc., New York).

In addition, the apolipoprotein or a fragment thereof may have conservative substitutions in 1, 2, 3, 4, 5, 6, 7, 8, 9, 10 or more amino acids in the amino acid sequence selected from SEQ ID NOs: 1 to 4, but is not limited thereto.

The term "conservative substitution" as used herein refers to the substitution of one amino acid with another having similar structural and/or chemical properties. The apolipoprotein or a fragment thereof may have, for example, one or more conservative substitutions while still retaining the biological activity of the native or a non-mutated signal peptide. Such amino acid substitutions may generally occur based on similarities in the polarity, charge, solubility, hydrophobicity, hydrophilicity and/or amphipathic nature of the residues. For example, positively charged (basic) amino acids include arginine, lysine, and histidine; negatively charged (acidic) amino acids include glutamic acid and aspartic acid; aromatic amino acids include phenylalanine, tryptophan, and tyrosine; and hydrophobic amino acids include alanine, valine, isoleucine, leucine, methionine, phenylalanine, tyrosine, and tryptophan. Additionally, amino acids can be classified into those with electrically charged side chains and those with uncharged side chains. Amino acids with charged side chains include aspartic acid, glutamic acid, lysine, arginine, and histidine, while amino acids with uncharged side chains may be further classified as nonpolar amino acids or polar amino acids. Nonpolar amino acids are glycine, alanine, valine, leucine, and isoleucine, methionine, and proline; whereas polar amino acids may include serine, threonine, cysteine, asparagine, and glutamine. Conservative substitutions with amino acids having such similar properties are expected to exhibit the same or similar activity.

The targeting protein may include a protein or a fragment thereof capable of binding to a protein (such as a ligand, receptor, or antigen) expressed on the surface of a target tissue or target cell.

In an embodiment, the targeting protein may be a protein that targets cancer tissue or cancer cells. Specifically, the targeting protein may include a ligand or a ligand-derived peptide capable of binding to a receptor expressed on the surface of cancer tissue or cancer cells. Additionally, the targeting protein may include a receptor or a receptor-derived peptide capable of binding to a ligand expressed on the surface of cancer tissue or cancer cells. Additionally, the targeting protein may include an antibody or a fragment thereof (antigen-binding fragment) capable of binding to a protein (antigen) expressed on the surface of cancer tissue or cancer cells.

The targeting protein may include one or more selected from the group consisting of PD-1, CTLA-4, CD28, ICOS, OX40, OX40L, GITR, GITRL, CD40, CD40L, CD47, SIRP-alpha, 4-1BB, 4-1BBL, B7-1, B7-2, B7-H1, B7-H2, B7-DC, CD80, CD160, BTLA, HVEM, DPP-4, NTCP, CD16, VEGF, VEGFR, Caveolin-1, TMIGD2, LIGHT, LAG3, CD27, NAM-1, CD96, 2B4, TIM-3, Adenosine A2a receptor, CEACAM1, DC-SIGN, CD200R, DR3, HVEM (CD270), CD137, TIGIT and CD112R, and may specifically include a PD-1 protein or a fragment thereof, or a SIRP-alpha protein or a fragment thereof.

In an embodiment, the PD-1 protein or a fragment thereof may include a polypeptide represented by the amino acid sequence of SEQ ID NO: 9, and specifically may consist of a polypeptide represented by the amino acid sequence of SEQ ID NO: 9. In addition, any amino acid sequence that exhibits a homology of 80% or more, specifically 90% or more, more specifically 95% or more, even more specifically 98% or more, and most specifically 99% or more to the above sequence, and that substantially exhibits the same or corresponding activity as the PD-1 protein or a fragment thereof, is included without limitation.

In an embodiment, the SIRP-alpha protein or a fragment thereof may include a polypeptide represented by the amino acid sequence of SEQ ID NO: 10, and specifically may consist of a polypeptide represented by the amino acid sequence of SEQ ID NO: 10. In addition, any amino acid sequence that exhibits a homology of 80% or more, specifically 90% or more, more specifically 95% or more, even more specifically 98% or more, and most specifically 99% or more to the above sequence, and that substantially exhibits the same or corresponding activity as the SIRP-alpha protein or a fragment thereof, is included without limitation.

The targeting protein may include an antibody or an antigen-binding fragment thereof capable of binding to one or more selected from the group consisting of PD-L1, PD-L2, MAGE-1, MAGE-2, MAGE-3, MAGE-12, BAGE, GAGE, NY-ESO-1, tyrosinase, TRP-1, TRP-2, gp100, MART-1, MCIR, Ig idotype, CDK4, Caspase-B, beta-catenin, CLA, BCR/ABL, mutated p21/ras, mutated p53, proteinase 3, WT1, MUC-1, Her2/neu, PAP, PSA, PSMA, G250, HPV E6/E7, EBV LMP2a, CEA, alpha-Fetoprotein, 5T4, CD47, and onco-trophoblast glycoprotein.

In the fusion protein, the apolipoprotein or a fragment thereof and the targeting protein or a fragment thereof may be linked via a linker, or may be directly connected. The linker is not particularly limited as long as it exhibits an effect of maintaining or improving the function and/or activity of the conjugated protein. As an example, the linker may include one or more amino acids or peptides. Additionally, the linker may include a peptide capable of binding to the Fc region of an antibody (Fc binding peptide, FcBP).

In an embodiment, the linker may include a peptide represented by the amino acid sequence of SEQ ID NO: 11, and may specifically consist of a peptide represented by the amino acid sequence of SEQ ID NO: 11. In addition, any amino acid sequence that exhibits a homology of 80% or more, specifically 90% or more, more specifically 95% or more, even more specifically 98% or more, and most specifically 99% or more to the above sequence, and that substantially exhibits the same or corresponding activity as the linker, is included without limitation.

In the fusion protein, the targeting protein or a fragment thereof may be linked to the N-terminus or C-terminus of an apolipoprotein (specifically, an ApoE protein) or a fragment thereof, and may be specifically linked to the N-terminus of an ApoE protein or a fragment thereof. Accordingly, the fusion protein may be linked sequentially in the order of targeting protein-apolipoprotein or a fragment thereof or 'targeting protein-linker-apolipoprotein or a fragment thereof' from the N-terminus.

In an embodiment, the fusion protein may include a polypeptide consisting of the following sequences (where the left end represents the N'-terminus and the right end represents the C'-terminus).
1) PD1 (SEQ ID NO: 9)-ApoE3 (SEQ ID NO: 1)
2) PD1 (SEQ ID NO: 9)-ApoE4 (SEQ ID NO: 2)
3) PD1 (SEQ ID NO: 9) - ApoE (151-299) (SEQ ID NO: 3)
4) PD1 (SEQ ID NO: 9) - ApoE (206-299) (SEQ ID NO: 4)
5) PD1 (SEQ ID NO: 9)-Linker (SEQ ID NO: 11)-ApoE3 (SEQ ID NO: 1)
6) PD1 (SEQ ID NO: 9)-Linker (SEQ ID NO: 11)-ApoE4 (SEQ ID NO: 2)
7) PD1 (SEQ ID NO: 9) - Linker (SEQ ID NO: 11) - ApoE (151-299) (SEQ ID NO: 3)
8) PD1 (SEQ ID NO: 9) - Linker (SEQ ID NO: 11) - ApoE (206-299) (SEQ ID NO: 4)
9) SIRPα (SEQ ID NO: 10)-ApoE3 (SEQ ID NO: 1)
10) SIRPα (SEQ ID NO: 10)-ApoE4 (SEQ ID NO: 2)
11) SIRPα (SEQ ID NO: 10)-ApoE (151-299) (SEQ ID NO: 3)
12) SIRPα (SEQ ID NO: 10)-ApoE (206-299) (SEQ ID NO: 4)
13) SIRPα (SEQ ID NO: 10)-Linker (SEQ ID NO: 11)-ApoE3 (SEQ ID NO: 1)
14) SIRPα (SEQ ID NO: 10)-Linker (SEQ ID NO: 11)-ApoE4 (SEQ ID NO: 2)
15) SIRPα (SEQ ID NO: 10)-Linker (SEQ ID NO: 11)-ApoE (151-299) (SEQ ID NO: 3)
16) SIRPα (SEQ ID NO: 10)-Linker (SEQ ID NO: 11)-ApoE (206-299) (SEQ ID NO: 4)

The fusion protein may be intended to enhance the delivery efficiency to a target cell or target tissue expressing a protein (such as an antigen, receptor or ligand) capable of binding to the targeting protein.

Another aspect is to provide a polynucleotide including a sequence encoding the fusion protein. The same part as described above are equally applicable to the polynucleotide.

The term "polynucleotide" as used herein refers to a polymer of deoxyribonucleotides or ribonucleotides existing in either single-stranded or doublestranded form. The term encompasses RNA genomic sequences, DNA (both gDNA and cDNA) and RNA sequences transcribed therefrom, and, unless otherwise specified, also includes analogs of naturally occurring polynucleotides.

The polynucleotide may consist of DNA, RNA or a modified form thereof, for example, cDNA, mRNA or a modified form thereof. When the polynucleotide is DNA or modified DNA, it may consist of A (Adenine), T (Thymine), G (Guanine), C (Cytosine) and/or modified forms thereof. When the polynucleotide is RNA or modified RNA, it may consist of A, U (Uridine), G, C and/or modified forms thereof. For example, the modified DNA or RNA may include one or more selected from the group consisting of 5-methylcytidine (5mC), N6-methyladenosine (m6A), 3,2'-O-dimethyluridine (m4U), 2-thiouridine (s2U), 2'-fluorouridine, pseudouridine, 2'-O-methyluridine (Um), 2'-deoxyuridine (2'dU), 4-thiouridine (s4U), 5-methyluridine (m5U), 2'-O-methyladenosine (m6A), N6,2'-O-dimethyladenosine (m6Am), N6,N6,2'-O-trimethyladenosine (m62Am), 2'-O-methylcytidine (Cm), 7-methylguanosine (m7G), 2'-O-methylguanosine (Gm), N2,7-dimethylguanosine (m2,7G), N2,N2,7-trimethylguanosine (m2,2,7G), and N1-methylpseudouridine.(m-2,2,7G) and N1-methyl-pseudouridine.

Accordingly, when the polynucleotide is in the form of RNA, the nucleotide sequence encoding the fusion protein described herein may be represented by replacing T with U. When the polynucleotide is in the form of modified DNA or modified RNA, the nucleotides A, T, G, C, and U in the nucleotide sequence encoding the fusion protein may each be replaced by their modified counterparts.

The polynucleotide encoding the fusion protein may undergo various modifications to the coding region within a range that does not change the amino acid sequence of the protein expressed from the coding region, taking into account the codons preferred in the organism in which the fusion protein is to be expressed due to the degeneracy of the codons. Therefore, the polynucleotide may be included without limitation as long as it is a polynucleotide sequence encoding the fusion protein. Additionally, a probe that may be prepared from a known sequence, for example, a sequence that hybridizes under stringent conditions with a complementary sequence to all or part of the polynucleotide sequence, and encodes a protein having the activity of the fusion protein, may be included without limitation.

The term "stringent conditions" refers to conditions that allows specific hybridization between polynucleotides. For example, the conditions may include those in which genes having high sequence homology, that is, genes having a homology of 40% or more, specifically 90% or more, more specifically 95% or more, even more specifically 97% or more, and 99% or more, are hybridized with each other, while genes having lower homology are not hybridized with each other. Alternatively, the conditions may include washing once, or specifically two to three times, at a salt concentration and temperature corresponding to the conventional Southern hybridization washing conditions of 60°C, 1×SSC, 0.1% SDS, specifically 60°C, 0.1×SSC, 0.1% SDS, and more specifically 68°C, 0.1×SSC, 0.1% SDS.

Hybridization requires that the two polynucleotides have complementary sequences, although mismatches between bases are possible depending on the stringency of hybridization. The term "complementary" refers to the relationship between nucleotide bases that may hybridize with each other. For example, in DNA, adenosine is complementary to thymine and cytosine is complementary to guanine. Accordingly, the present disclosure may also include isolated polynucleotide fragments that are complementary to the entire sequence as well as substantially similar polynucleotide sequences.

The sequence encoding the fusion protein may include a sequence encoding an apolipoprotein or a fragment thereof and a sequence encoding a targeting protein.

In an embodiment, the sequence encoding the apolipoprotein may include a polynucleotide represented by the nucleotide sequence of SEQ ID NO: 5 or SEQ ID NO: 6, and specifically may consist of a polynucleotide represented by the nucleotide sequence of SEQ ID NO: 5 or SEQ ID NO: 6. In addition, any nucleotide sequence that exhibits a homology of 80% or more, specifically 90% or more, more specifically 95% or more, even more specifically 98% or more, and most specifically 99% or more to the above sequence, and that substantially encodes a protein having the same or corresponding activity as the apolipoprotein, is included without limitation.

In an embodiment, the sequence encoding the fragment of the apolipoprotein may include a polynucleotide represented by the nucleotide sequence of SEQ ID NO: 7 or SEQ ID NO: 8, and specifically may be composed of a polynucleotide represented by the nucleotide sequence of SEQ ID NO: 7 or SEQ ID NO: 8. In addition, any nucleotide sequence that exhibits a homology of 80% or more, specifically 90% or more, more specifically 95% or more, even more specifically 98% or more, and most specifically 99% or more to the above sequence, and that substantially encodes a protein having the same or corresponding activity as the apolipoprotein, is included without limitation.

Another aspect provides an expression vector including the polynucleotide. The same part as described above are equally applicable to the expression vector.

The term "expression vector" as used herein refers to a recombinant vector capable of expressing a target protein when introduced into a suitable host cell or organism, and includes a genetic construct containing essential regulatory elements operably linked to enable expression of a gene insert. The term "operably linked" means that a nucleic acid expression control sequence is functionally connected to a nucleic acid sequence encoding a desired protein so that they may perform their general functions. The operational linkage with the recombinant vector may be prepared using a genetic recombination technique well known in the art, and the site-specific DNA cleavage and linkage may be easily accomplished using enzymes generally known in the art.

A suitable expression vector of the present disclosure may include a signal sequence for membrane targeting or secretion in addition to expression control elements such as a promoter, initiation codon, termination codon, polyadenylation signal, and enhancer. The initiation and termination codons are generally considered to be part of the nucleotide sequence encoding the target protein, must be functional in a subject when the genetic construct is administered, and should be in-frame with the coding sequence. General promoters may be constitutive or inducible. For prokaryotic cells, examples include lac, tac, T3, and T7 promoters. For eukaryotic cells, examples include promoters derived from Simian Virus 40 (SV40), Mouse Mammary Tumor Virus (MMTV), Human Immunodeficiency Virus (HIV) (for example, the long terminal repeat (LTR) promoter of HIV), Moloney virus, Cytomegalovirus (CMV), Epstein-Barr Virus (EBV), and Rous Sarcoma Virus (RSV), as well as promoters derived from β-actin, human hemoglobin, human muscle creatine, and human metallothionein, but are not limited thereto.

Additionally, the expression vector may include a selectable marker for selecting host cells containing the vector. The selectable marker is used to identify cells transformed with a vector, and markers that confer a selectable phenotype such as drug resistance, auxotrophy, resistance to cytotoxic agents, or expression of surface proteins may be used. In an environment treated with a selective agent, only cells expressing the selectable marker may survive, thereby allowing selection of transformed cells. Additionally, if the vector is a replicable expression vector, it may include a replication origin, which is a specific nucleic acid sequence at which replication is initiated.

Various types of vectors, such as plasmids, viruses, and cosmids, may be used as recombinant expression vectors for inserting foreign genes. The type of recombinant vector is not particularly limited as long as it has the function of expressing a desired gene and producing a desired protein in various host cells of prokaryotic and eukaryotic cells. However, a vector that may produce a large amount of a foreign protein in a form similar to that in the natural state while having a promoter that exhibits strong activity and a strong expression ability may be used.

To express the protein of the present disclosure, various combinations of hosts and vectors may be used. Expression vectors suitable for eukaryotic hosts may include, but are not limited to, expression control sequences derived from SV40, bovine papillomavirus, adenovirus, adeno-associated virus, cytomegalovirus, and retrovirus. Expression vectors that may be used in bacterial hosts include, but are not limited to, bacterial plasmids obtained from *Escherichia coli,* including pET21a, pET, pRSET, pBluescript, pGEX2T, pUC vectors, col E1, pCR1, pBR322, pMB9 or derivatives thereof; plasmids having a broader host range such as RP4; phage DNA such as phage lambda derivatives such as λgt10, λgt11 or NM989; and other DNA phages such as M13 or filamentous single-stranded DNA phages. In yeast cells, 2µ plasmids or derivatives thereof may be used, while in insect cells, vectors such as pVL941 may be employed.

The cell, for example, a eukaryotic cell, may be a yeast, fungal, protozoa, plant, higher plant, insect, or amphibian cell, or a mammalian cell such as CHO, HeLa, HEK293, and COS-1. For example, the cell may be a cultured cell (in vitro), a graft cell, a primary cultured cell (in vitro and ex vivo), or an in vivo cell, and may also be a mammalian cell, including humans, which are commonly used in the art. Additionally, the organism may be a yeast, a mold, a protozoan, a plant, a higher plant, an insect, an amphibian, or a mammal.

Another aspect provides a composition for targeting or a use for targeting including the fusion protein. The same part as described above are equally applicable to the composition or use.

The fusion protein or a composition including the same may be for targeting or improving the targeting efficiency of a lipid-based carrier, specifically for targeting or improving the targeting efficiency of the carrier to a specific cell or tissue, and more specifically for targeting or improving the targeting efficiency of the carrier to a target cell or target tissue that expresses a protein (such as an antigen, receptor, or ligand) capable of binding to the targeting protein included in the fusion protein.

In an embodiment, the apolipoprotein (specifically, ApoE) or a fragment thereof in the fusion protein may be bound to the lipid portion of the lipid-based carrier.

Another aspect provides a targeting carrier including the fusion protein and a lipid-based carrier. The same part as described above are equally applicable to the carrier.

The lipid-based carrier may include one or more selected from the group consisting of a lipid nanoparticle (LNP), a liposome, a nano-structured lipid carrier (NLC), a lipid microemulsion (LME), a lipid nanoemulsion (LNE), a solid lipid nanoparticle (SLN), a self-emulsifying delivery system (SEDDS), a lipid sphere (Liposphere), a solid lipid microparticle (SLM), a lipid drug conjugate (LDC), an exosome, a niosome, a pharmacosome, a phytosome, an ethosome, and a herbosome.

The lipid-based carrier may include one or more selected from the group consisting of phospholipids, sterols, PEG (polyethylene glycol)-lipids, and ionizable lipids. Additionally, the ionized lipid may be a cationic lipid, and the sterol may be cholesterol.

In the targeting carrier, the fusion protein may be linked or conjugated to a lipid-based carrier. Specifically, a region of the fusion protein including the apolipoprotein (specifically, the ApoE protein) or a fragment thereof may be linked or bound to a lipid that constitutes the lipid-based carrier.

The targeting protein or a fragment thereof included in the fusion protein of the targeting carrier may bind to a cell or tissue to be targeted, and through the mechanism, the targeting carrier of the present disclosure may be effectively delivered or moved to the cell or tissue to be targeted.

The targeting carrier may be for inducing targeting to a target cell or target tissue expressing a protein (such as an antigen, receptor or ligand) capable of binding to the targeting protein included in the fusion protein, or for improving targeting efficiency.

The targeting carrier may target cancer, and may specifically enhance delivery efficiency to cancer tissues or cancer cells.

The targeting carrier may additionally include a therapeutic drug of interest intended for delivery. Accordingly, the targeting carrier may be a drug carrier.

The targeting carrier or drug carrier may be for inducing drug delivery to a cell or tissue (target cell or target tissue) that expresses a protein (such as an antigen, receptor or ligand) capable of binding to the targeting protein included in the fusion protein, or for improving drug delivery efficiency.

The term "drug carrier" or "drug delivery system (DDS)" as used herein refers to a series of technologies or materials that control the delivery and release of pharmacologically active substances into cells, tissues, organs, and systems to achieve optimal efficacy by using various physicochemical technologies, and refers to a technology that optimizes drug treatment by designing a formulation to efficiently deliver the required amount of drugs while minimizing side effects of existing drugs and maximizing efficacy and effectiveness.

Another aspect provides a composition for drug delivery including the targeting carrier. The same parts as described above are equally applicable to the composition.

The composition may further include a drug of interest.

The composition may deliver or transport the drug of interest to target cells or tissues to be targeted through the targeting carrier.

The drug is not particularly limited as long as it may exhibit pharmacological activity and therapeutic effects in tissues and/or in vivo as an active ingredient, and specifically, it may be a pharmaceutical or bioactive substance. For example, it may include one or more selected from the group consisting of: proteins (including enzymes, ligands, antibodies, and the like); nucleic acids (including compounds, siRNA, microRNA, mRNA, plasmids, genes, and the like); aptamers; genetic materials; lipids; carbohydrates; dyes; photosensitizers; anticancer agents; antibiotics; chemical compounds; and combinations thereof, but is not limited thereto.

In an embodiment, the drug may be a nucleic acid, and the nucleic acid may include one or more selected from the group consisting of single-stranded siRNA, doublestranded siRNA, rRNA, RNA, DNA, cDNA, plasmid, aptamer, mRNA, tRNA, IncRNA, piRNA, circRNA, saRNA, antisense oligonucleotide, shRNA, miRNA, ribozyme, PNA, microRNA, ASO, gapmer, aptamer, antagomir, agomir, and DNAzyme.

Another aspect provides a pharmaceutical composition including the targeting carrier and a drug of interest. The same parts as described above are equally applicable to the composition.

The targeting carrier may be for delivering a drug of interest. Accordingly, the pharmaceutical composition may be a composition for drug delivery.

The drug of interest may be a drug for treating, preventing or improving cancer, and specifically, may be an anticancer agent or an anticancer adjuvant agent.

The pharmaceutical composition may be for treating or preventing cancer. The cancer may include one or more selected from the group consisting of liver cancer, lung cancer, pancreatic cancer, non-small cell lung cancer, colon cancer, bone cancer, skin cancer, head or neck cancer, cutaneous or intraocular melanoma, uterine cancer, ovarian cancer, rectal cancer, gastric cancer, perianal cancer, colorectal cancer, breast cancer, fallopian tube carcinoma, endometrial carcinoma, cervical carcinoma, vaginal carcinoma, vulvar carcinoma, Hodgkin's disease, esophageal cancer, small intestine cancer, endocrine gland cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureter cancer, renal cell carcinoma, renal pelvic carcinoma, central nervous system (CNS) tumor, primary CNS lymphoma, spinal tumor, brainstem glioma, and pituitary adenoma.

The pharmaceutical composition may include a pharmaceutically acceptable carrier. The term "pharmaceutically acceptable carrier" refers to a carrier or diluent that does not stimulate the organism and does not inhibit the biological activity and properties of the administered compound. Herein, the term "pharmaceutically acceptable" means that the substance does not inhibit the activity of the active ingredient and does not possess toxicity greater than that which is acceptable for the intended subject of administration (prescription). Any carrier conventionally used in the art and pharmaceutically acceptable may be employed in the pharmaceutical composition. Nonlimiting examples of such carrier include lactose, dextrose, maltodextrin, sucrose, sorbitol, mannitol, xylitol, erythritol, maltitol, glycerol, ethanol, starch, acacia gum, alginate, gelatin, calcium phosphate, calcium silicate, cellulose, methyl cellulose, microcrystalline cellulose, polyvinyl pyrrolidone, water, saline solution, sterile water, Ringer's solution, buffered saline, albumin injection solution, methylhydroxybenzoate, propylhydroxybenzoate, talc, magnesium stearate, or mineral oil. These carriers may be used alone or in combination of two or more thereof. The pharmaceutical composition may be prepared as an oral formulation or a parenteral formulation depending on the route of administration by a conventional method known in the art, including the active ingredient and a pharmaceutically acceptable carrier. The pharmaceutical composition may be formulated and used in the form of oral formulations such as powders, granules, tablets, capsules, suspensions, emulsions, syrups, or aerosols, or in the form of topical preparations, suppositories, or sterile injectable solutions, according to a conventional method.

When formulating the pharmaceutical composition, it may be prepared using a diluent or excipient such as fillers, extenders, binders, wetting agents, disintegrants, or surfactants that are commonly used in the art, but is not limited thereto.

When the pharmaceutical composition is prepared as an oral dosage form, it may be prepared in the form of powder, granules, tablets, pills, sugar-coated tablets, capsules, liquids, gels, syrups, suspensions, or wafers, according to methods known in the art, together with a suitable carrier. Examples of pharmaceutically acceptable carriers include sugars such as lactose, glucose, sucrose, dextrose, sorbitol, mannitol, and xylitol; starches such as corn starch, potato starch, and wheat starch; celluloses such as cellulose, methyl cellulose, ethyl cellulose, sodium carboxymethyl cellulose, and hydroxypropyl methyl cellulose; polyvinylpyrrolidone, water, methyl hydroxybenzoate, propyl hydroxybenzoate, magnesium stearate, mineral oil, malt, gelatin, talc, polyols, and vegetable oils. In case of formulation, the formulation may include diluents and/or excipients such as fillers, extenders, binders, wetting agents, disintegrants, and surfactants, as needed.

When the pharmaceutical composition is prepared as a parenteral formulation, it may be formulated into the form of an injection, a transdermal administration agent, a nasal inhalant, and a suppository, according to methods known in the art, together with a suitable carrier. For injectable formulations, suitable carriers include sterile water, ethanol, polyols such as glycerol or propylene glycol, or mixtures thereof; preferably, Ringer's solution, phosphate-buffered saline (PBS) containing triethanolamine, sterile water for injection, or isotonic solutions such as 5% dextrose may be used. When formulated as a transdermal preparation, the pharmaceutical composition may be prepared in the form of an ointment, cream, lotion, gel, external solution, paste, liniment, or aerosol. In the case of a nasal inhalation formulation, the composition may be formulated as an aerosol spray using suitable propellants such as dichlorofluoromethane, trichlorofluoromethane, dichlorotetrafluoroethane, or carbon dioxide. When formulated as a suppository, suitable bases may include witepsol, tween 61, polyethylene glycols, cacao butter, laurin oil, polyoxyethylene sorbitan fatty acid esters, polyoxyethylene stearates, or sorbitan fatty acid esters.

The pharmaceutical composition may be administered in a pharmaceutically effective amount. The term "pharmaceutically effective amount" refers to an amount sufficient to treat or prevent a disease with a reasonable benefit-to-risk ratio applicable to medical treatment or prevention. The effective dosage level may be determined based on factors such as the severity of the disease, the activity of the drug, the age, body weight, health condition, and sex of the patient, the patient's sensitivity to the drug, the administration time, administration route, excretion rate, treatment duration, the combination or concurrent use of the composition of the present disclosure with other drugs, and other factors well known in the medical field. The pharmaceutical composition of the present disclosure may be administered alone or in combination with other agents known to exhibit therapeutic effects on the relevant disease. It is important to administer an amount that achieves maximum efficacy with the minimum dose without causing side effects, taking all of the above factors into consideration.

The dosage of the pharmaceutical composition may be determined by those skilled in the art, considering factors such as the intended use, severity of the condition, age, weight, sex, medical history of the patient, and the type of active ingredient used. For example, the pharmaceutical composition of the present disclosure may be administered to an adult in an amount of about 0.1 ng to about 1,000 mg/kg, preferably about 1 ng to about 100 mg/kg. The administration frequency of the composition is not particularly limited, but the composition may be administered once daily, or multiple times per day by dividing the total dose. Neither the dosage nor the frequency of administration should be construed as limiting the scope of the present disclosure.

Another aspect provides a method of delivering a drug of interest to a subject, the method including administering to the subject the targeting carrier, the drug carrier, or the composition for drug delivery. The same parts as described above also are equally applicable to the composition.

The term "subject" as used herein may include, without limitation, mammals including dogs, cats, rats, livestock, and humans, birds, reptiles, and aquaculture fish.

The method may be used to deliver a drug of interest to cells, tissues, and/or organs within a subject that express a ligand, receptor, and/or antigen capable of binding to the targeting protein of the present disclosure. Specifically, the method may be used to deliver a desired drug to cancer cells and/or cancer tissues.

### Advantageous Effects

According to an aspect of the fusion protein, it was confirmed that lipid-based carriers containing the fusion protein exhibited significantly enhanced targeting efficiency. Therefore, by using the fusion protein, an active ingredient may be effectively delivered to a desired target.

### Description of Drawings

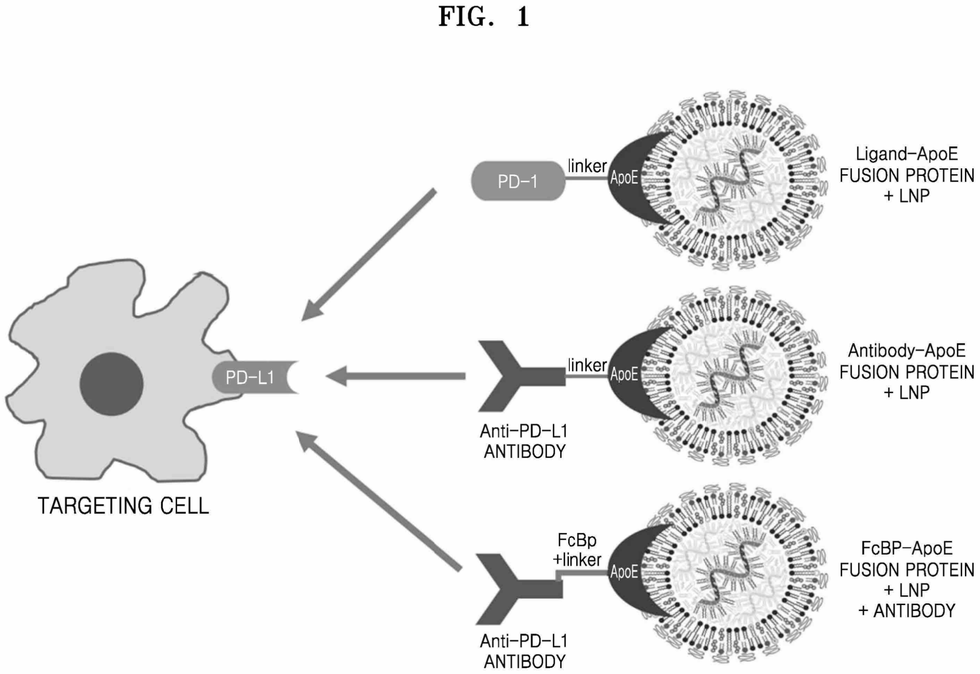

FIG. 1 is a schematic diagram showing a lipid-based carrier using a fusion protein of the present disclosure.
FIG. 2 is a diagram showing the description of each region of an ApoE protein.
FIG. 3 is a drawing showing the results of confirming the targeting delivery efficacy of lipid nanoparticles produced using a fusion protein including an ApoE protein of the present disclosure.
FIG. 4 is a diagram showing the structure and domains of an ApoE4 protein.
FIGS. 5 to 7 are drawings showing the results of confirming the targeting delivery efficacy of lipid nanoparticles produced using a fusion protein including an ApoE protein fragment of the present disclosure.

### Mode for Invention

The present disclosure will now be described in further detail with reference to the following Examples. However, it should be understood that these Examples are provided for illustrative purposes only and are not intended to limit the scope of the present disclosure in any way.

### Example 1: Evaluation of Targeting Efficiency of a Carrier Including an ApoE Protein-Containing Fusion Protein

The present disclosure relates to a fusion protein including an apolipoprotein, such as ApoE, and a targeting protein, and a targeting carrier including the same. To evaluate the targeting delivery efficiency of a lipid-based carrier containing the fusion protein of the present disclosure, the following experiment was performed.

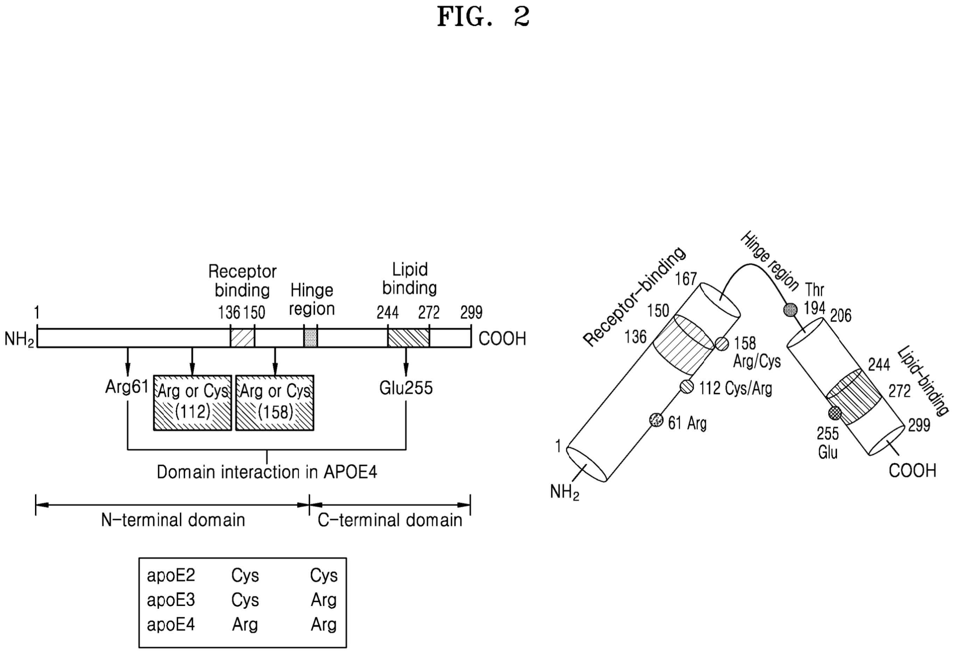

Specifically, fusion proteins including the ApoE protein, such as ApoE3 or ApoE4, and a targeting protein were prepared, wherein the targeting protein of the fusion protein was, in an example, the PD1 protein (SEQ ID NO: 9). Additionally, the fusion proteins were constructed such that the ApoE protein and PD1 protein were linked either in the order of ApoE-PD1 (from the N-terminus) or in the reverse order, PD1-ApoE. Meanwhile, the structure and domains of the ApoE protein are illustrated in FIG. 2, and the amino acid and nucleotide sequences of ApoE3 and ApoE4 are shown in Tables 1 and 2, respectively (wherein, in Table 2, "U" in the nucleotide sequence is replaced with "T" when the sequence represents DNA).

**Table 1**

| Protein | Amino Acid Sequence | Seque nce ID No. |
|---|---|---|
| ApoE3 | KVEQAVETEPEPELRQQTEWQSGQRWELALGRFWDYLRW VQTLSEQVQEELLSSQVTQELRALMDETMKELKAYKSELE EQLTPVAEETRARLSKELQAAQARLGADMEDVCGRLVQY RGEVQAMLGQSTEELRVRLASHLRKLRKRLLRDADDLQK RLAVYQAGAREGAERGLSAIRERLGPLVEQGRVRAATVGS LAGQPLQERAQAWGERLRARMEEMGSRTRDRLDEVKEQV AEVRAKLEEQAQQIRLQAEAFQARLKSWFEPLVEDMQRQ WAGLVEKVQAAVGTSAAPVPSDNH | 1 |
| ApoE4 | KVEQAVETEPEPELRQQTEWQSGQRWELALGRFWDYLRW VQTLSEQVQEELLSSQVTQELRALMDETMKELKAYKSELE EQLTPVAEETRARLSKELQAAQARLGADMEDVCGRLVQY | 2 |
| | RGEVQAMLGQSTEELRVRLASHLRKLRKRLLRDADDLQK RLAVYQAGAREGAERGLSAIRERLGPLVEQGRVRAATVGS LAGQPLQERAQAWGERLRARMEEMGSRTRDRLDEVKEQV AEVRAKLEEQAQQIRLQAEAFQARLKSWFEPLVEDMQRQ WAGLVEKVQAAVGTSAAPVPSDNH | |

**Table 2**

| Protein | Nucleotide Sequence (mRNA Sequence) | Seque nce ID No. |
|---|---|---|
| ApoE3 | AAA GTG GAG CAA GCT GTG GAA ACG GAG CCA GAG CCC GAA TTG CGG CAA CAA ACT GAG TGG CAG AGC GGT CAA AGG TGG GAA CTC GCC CTT GGT AGA TTT TGG GAT TAT TTG CGG TGG GTC CAA ACA CTC AGC GAG CAG GTA CAA GAG GAA CTC CTC TCC TCC CAG GTT ACA CAG GAG CTG AGG GCT TTG ATG GAT GAG ACA ATG AAG GAG CTG AAG GCT TAT AAA AGT GAA TTG GAA GAA CAG CTT ACG CCT GTT GCT GAA GAG ACC AGA GCA AGA CTG AGC AAG GAG CTT CAA GCT GCC CAG GCA CGA CTT GGG GCC GAC ATG GAA GAC GTT TGT GGG CGC CTT GTC CAA TAC AGA GGA GAG GTG CAG GCG ATG CTG GGC CAG TCT ACA GAG GAG CTC CGG GTT CGG CTT GCG TCA CAC CTC AGA AAA TTG AGG AAG CGG CTC CTG CGC GAT GCT GAC GAT CTT CAG AAA CGA CTG GCA GTA TAT CAG GCC GGA GCA AGA GAG GGT GCC GAA CGC GGG TTG AGC GCC ATC CGG GAA CGC TTG GGT CCC CTT GTT GAA CAA GGG CGC GTT AGA GCC GCA ACC GTT GGG AGT TTG GCT GGT CAG CCT CTC CAG GAG CGA GCG CAA GCA TGG GGT GAG AGG TTG CGA GCC CGG ATG GAG GAA | 5 |
| | ATG GGA TCT AGG ACC CGG GAC CGC CTG GAT GAG GTT AAG GAA CAA GTC GCT GAG GTA CGC GCG AAA CTT GAG GAA CAA GCA CAG CAG ATT AGA CTT CAG GCA GAG GCA TTT CAG GCG CGG CTT AAG AGT TGG TTC GAG CCG CTC GTG GAA GAT ATG CAG CGC CAA TGG GCA GGA CTC GTC GAA AAA GTT CAG GCA GCT GTC GGG ACG AGT GCA GCC CCT GTC CCA TCA GAC AAT CAC | |
| ApoE4 | AAA GTG GAG CAA GCT GTG GAA ACG GAG CCA GAG CCC GAA TTG CGG CAA CAA ACT GAG TGG CAG AGC GGT CAA AGG TGG GAA CTC GCC CTT GGT AGA TTT TGG GAT TAT TTG CGG TGG GTC CAA ACA CTC AGC GAG CAG GTA CAA GAG GAA CTC CTC TCC TCC CAG GTT ACA CAG GAG CTG AGG GCT TTG ATG GAT GAG ACA ATG AAG GAG CTG AAG GCT TAT AAA AGT GAA TTG GAA GAA CAG CTT ACG CCT GTT GCT GAA GAG ACC AGA GCA AGA CTG AGC AAG GAG CTT CAA GCT GCC CAG GCA CGA CTT GGG GCC GAC ATG GAA GAC GTT TGT GGG CGC CTT GTC CAA TAC AGA GGA GAG GTG CAG GCG ATG CTG GGC CAG TCT ACA GAG GAG CTC CGG GTT CGG CTT GCG TCA CAC CTC AGA AAA TTG AGG AAG CGG CTC CTG CGC GAT GCT GAC GAT CTT CAG AAA CGA CTG GCA GTA TAT CAG GCC GGA GCA AGA GAG GGT GCC GAA CGC GGG TTG AGC GCC ATC CGG GAA CGC TTG GGT CCC CTT GTT GAA CAA GGG CGC GTT AGA GCC GCA ACC GTT GGG AGT TTG GCT GGT CAG CCT CTC CAG GAG CGA GCG CAA GCA TGG GGT GAG AGG TTG CGA GCC CGG ATG GAG GAA ATG GGA TCT AGG ACC CGG GAC CGC CTG GAT GAG | 6 |
| | GTT AAG GAA CAA GTC GCT GAG GTA CGC GCG AAA CTT GAG GAA CAA GCA CAG CAG ATT AGA CTT CAG GCA GAG GCA TTT CAG GCG CGG CTT AAG AGT TGG TTC GAG CCG CTC GTG GAA GAT ATG CAG CGC CAA TGG GCA GGA CTC GTC GAA AAA GTT CAG GCA GCT GTC GGG ACG AGT GCA GCC CCT GTC CCA TCA GAC AAT CAC | |

The fusion protein produced as described above was mixed with lipid nanoparticles to prepare lipid nanoparticles conjugated with the fusion protein (targeting carrier). Meanwhile, in order to evaluate the targeting efficacy of the lipid nanoparticles, lipid nanoparticles loaded with mRNA of Firefly Luciferase were used as an example of an active ingredient. To prepare the luciferase-loaded lipid nanoparticles, ionizable lipids, non-cationic lipids, sterols, and pegylated lipids (PEG) were mixed. Next, the mRNA to be encapsulated in the lipid nanoparticles was included in a citric acid buffer solution between pH 3.0 and pH 3.5 at a concentration of N/P ratio between 4 and 20. The lipid mixture and the mRNA solution were then combined at a volume ratio of 1:3 and mixed at a flow rate of 12.0 mL/min. For the preparation of the lipid nanoparticles, a NanoAssemblr Ignite (model NIN0001, Precision Nanosystems) instrument was used. The lipid and mRNA solutions mixed through the instrument underwent ethanol removal by ultrafiltration using an Amicon 100 kDa membrane (Millipore, UFC810024).

To verify whether the lipid nanoparticles prepared as described above effectively encapsulated firefly luciferase mRNA, the RiboGreen Assay Kit (Invitrogen, R11490) was used to measure the mRNA content. Specifically, the solution provided in the Ribogreen kit is a fluorescent substance that stains nucleic acids, and may measure the encapsulation efficiency within lipid nanoparticles by measuring the amount of RNA in the solution. To stain the mRNA inside the lipid nanoparticles, the nanoparticles were disrupted with a 2% Triton X-100 solution to release the mRNA. The resulting lysed lipid nanoparticle solution was then mixed with the RiboGreen solution, and fluorescence was measured using a microplate reader at excitation 485 nm and emission 530 nm. As a result, it was confirmed that the lipid nanoparticles exhibited excellent encapsulation efficiency (see Table 3).

**Table 3**

| **No.** | **Sample ID** | **Encapsulation Efficiency (%)** | **Total mRNA Conc. (ug/ml)** | **Encapsulated mRNA Conc. (ug/ml)** |
|---|---|---|---|---|
| 1 | Firefly Luciferase mRNA-LNP | 79.3 | 95.9 | 76.0 |

Next, to evaluate the targeting efficiency of the lipid nanoparticles containing the fusion protein, the targeting carrier prepared as described above was applied to 293T cells (control) or 293T-mPDL1 cells (293T cells expressing mouse PDL1 on the surface). After 24 hours, the cells were harvested, and a luciferase assay was performed using the cell lysates to assess the delivery efficiency of the targeting carrier. In addition, to compare the delivery efficiency of the fusion proteins, lipid nanoparticles containing PD1, ApoE3, or ApoE4 individually were prepared and used as controls. The luciferase assay was performed using the Promega, Dual-Luciferase^{®} Reporter Assay System, #E1910 kit after adjusting the protein concentration in the cell lysate to be the same across samples.

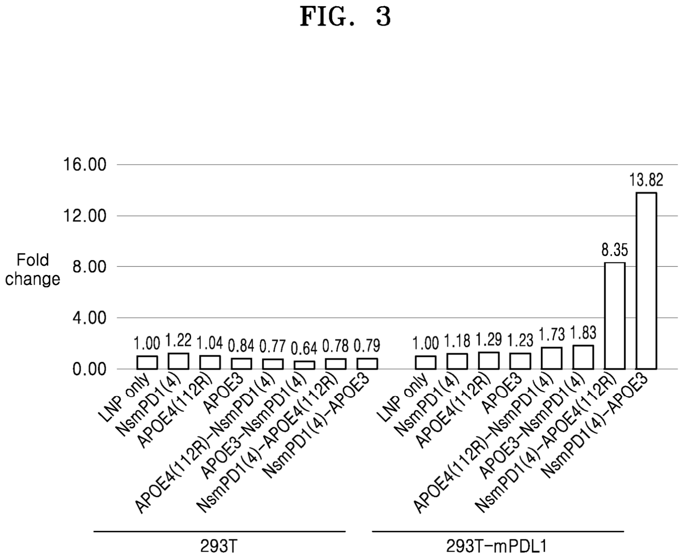

As a result, in the control 293T cells, results similar to those obtained when lipid nanoparticles were treated alone were observed, regardless of the type of protein linked to the lipid nanoparticles. In contrast, in 293T-mPDL1 cells expressing PDL1 on the surface that bind to the targeting protein PD1, it was confirmed that the delivery efficiency of LNPs produced using a fusion protein including ApoE3 and PD1 or including ApoE4 and PD1 was significantly increased compared to LNPs produced using only LNPs or LNPs produced by linking only PD1, ApoE3, or ApoE4 (FIG. 3). In addition, it was confirmed that when using PD1-ApoE3 and PD1-ApoE4, in which the targeting protein (PD1) is linked to the N-terminus of ApoE among the fusion proteins, the delivery efficiency was significantly improved compared to when using ApoE3-PD1 and ApoE4-PD1 (in which the targeting protein is linked to the C-terminus of ApoE).

Based on these results, it may be seen that a fusion protein including ApoE and a targeting protein may improve the targeting efficacy of lipid nanoparticles, and in particular, a fusion protein in which a targeting protein is linked to the N-terminus of ApoE may significantly improve the delivery efficiency of lipid nanoparticles.

### Example 2: Evaluation of the Targeting Efficiency of a Carrier Including Fusion Protein Containing an ApoE Variant

To improve the targeting efficiency of the fusion protein containing the ApoE protein, the following experiment was conducted.

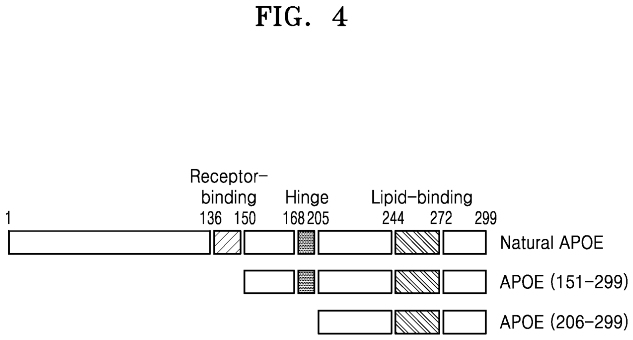

Specifically, to enhance targeting efficiency, two ApoE variants were generated by deleting portions of the N-terminal region of the ApoE protein (see FIG. 4). Fusion proteins including each ApoE variant and a targeting protein were then constructed. The first variant consists of the 151st to 299th amino acids of the ApoE protein, excluding the 1st to 150th amino acids thereof, and the second variant consists of the 206th to 299th amino acids of the ApoE protein, excluding the 1st to 205th amino acids thereof. The amino acid and nucleotide sequences of the first and second variants are shown respectively in Tables 4 and 5 (in Table 5, when the nucleotide sequence corresponds to DNA, "U" is replaced with "T").

**Table 4**

| Protein | Amino Acid Sequence | Seque nce ID No. |
|---|---|---|
| First variant [ApoE(151-299) | DADDLQKRLAVYQAGAREGAERGLSAIRERLGPLVE QGRVRAATVGSLAGQPLQERAQAWGERLRARMEEM GSRTRDRLDEVKEQVAEVRAKLEEQAQQIRLQAEAF QARLKSWFEPLVEDMQRQWAGLVEKVQAAVGTSAA PVPSDNH | 3 |
| Second variant [ApoE(206-299) | RAQAWGERLRARMEEMGSRTRDRLDEVKEQVAEV RAKLEEQAQQIRLQAEAFQARLKSWFEPLVEDMQR QWAGLVEKVQAAVGTSAAPVPSDNH | 4 |

**Table 5**

| Protein | Nucleotide Sequence (mRNA Sequence) | Seque nce ID No. |
|---|---|---|
| First variant [ApoE(151-299) | GAU GCU GAC GAU CUU CAG AAA CGA CUG GCA GUA UAU CAG GCC GGA GCA AGA GAG GGU GCC GAA CGC GGG UUG AGC GCC AUC CGG GAA CGC UUG GGU CCC CUU GUU GAA CAA GGG CGC GUU AGA GCC GCA ACC GUU GGG AGU UUG GCU GGU CAG CCU CUC CAG GAG CGA GCG CAA GCA UGG GGU GAG AGG UUG CGA GCC CGG AUG GAG GAA AUG GGA UCU AGG ACC CGG GAC CGC CUG GAU GAG GUU AAG GAA CAA GUC GCU GAG GUA CGC GCG AAA CUU GAG GAA CAA GCA CAG CAG AUU AGA CUU CAG GCA GAG GCA UUU CAG GCG CGG CUU AAG AGU UGG UUC GAG CCG CUC GUG GAA GAU AUG CAG CGC CAA UGG GCA GGA CUC GUC GAA AAA GUU CAG GCA GCU GUC GGG ACG AGU GCA GCC CCU GUC CCA UCA GAC AAU CAC | 7 |
| Second variant [ApoE(206-299) | GAU GCU GAC GAU CUU CAG AAA CGA CUG GCA GUA UAU CAG GCC GGA GCA AGA GAG GGU GCC GAA CGC GGG UUG AGC GCC AUC CGG GAA CGC UUG GGU CCC CUU GUU GAA CAA GGG CGC GUU AGA GCC GCA ACC GUU GGG AGU UUG GCU GGU CAG CCU CUC CAG GAG CGA GCG CAA GCA UGG GGU GAG AGG UUG CGA GCC CGG AUG GAG GAA AUG GGA UCU AGG ACC CGG GAC CGC CUG GAU GAG GUU AAG GAA CAA GUC GCU GAG GUA CGC GCG AAA CUU GAG GAA CAA GCA CAG CAG AUU AGA CUU CAG GCA GAG GCA UUU CAG GCG CGG CUU AAG AGU UGG UUC GAG CCG CUC GUG GAA GAU AUG CAG CGC CAA UGG GCA GGA CUC GUC | 8 |
| | GAA AAA GUU CAG GCA GCU GUC GGG ACG AGU GCA GCC CCU GUC CCA UCA GAC AAU CAC | |

The fusion protein containing the ApoE protein variant was mixed with luciferase-loaded lipid nanoparticles in the same manner as in Example 1 to produce a lipid nanoparticle (targeting carrier) to which the fusion protein was linked. After treatment with 293T cells (control) or 293T-mPDL1 cells, a luciferase assay was performed with cell lysate 24 hours later to evaluate the delivery efficiency of the targeting carrier. In addition, to evaluate the delivery efficiency of the fusion protein, lipid nanoparticles linked to PD1, ApoE3, ApoE4, ApoE(151-299), or ApoE(206-299) proteins were prepared and used as a control.

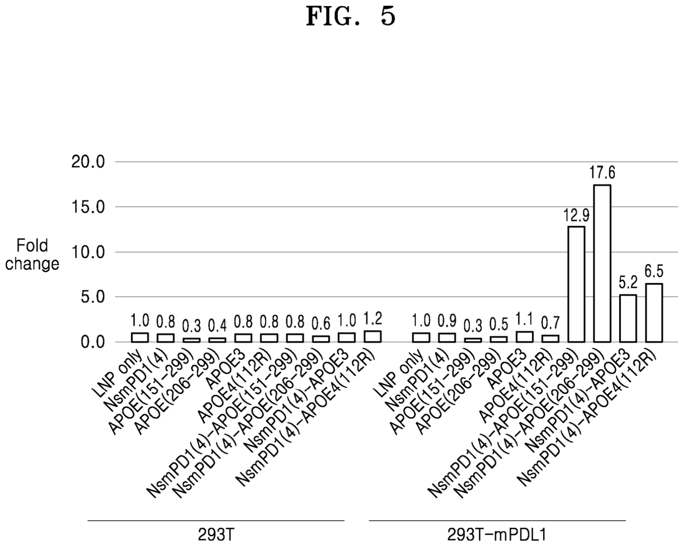

As a result, in 293T-mPDL1 cells expressing PDL1 on the surface that may bind to the targeting protein PD1, it was confirmed that the delivery efficiency of LNPs produced using a fusion protein including ApoE3 and PD1 or including ApoE4 and PD1 was improved compared to LNPs produced using only LNPs or including only PD1, ApoE3, ApoE4, ApoE(151-299) or ApoE(206-299). In addition, the LNPs containing the fusion proteins including ApoE(151-299) with PD1, or ApoE(206-299) with PD1-both having a partially truncated N-terminus of the ApoE protein-exhibited further improved delivery efficiency, and in particular, the LNP containing the fusion protein including ApoE(206-299) and PD1 showed the highest delivery efficiency (FIG. 5).

Next, to confirm whether the LNP linked to the fusion protein including the ApoE protein variant and the targeting protein exhibits excellent targeting efficacy in cells expressing human PDL1 (hPDL1) on the surface, the targeting carrier manufactured above was applied to 293T cells (control) or 293T-hPDL1 cells (293T expressing hPDL1 on the surface), and then 24 hours later, a luciferase assay was performed with cell lysates to evaluate the delivery efficiency of the targeting carrier.

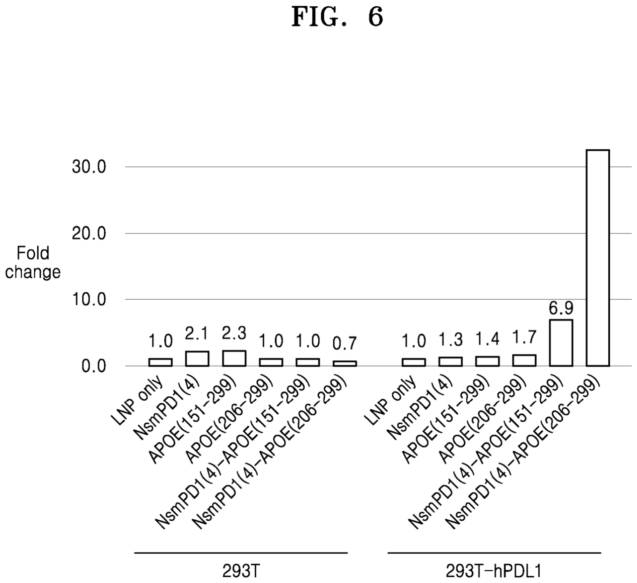

As a result, it was confirmed that the delivery efficiency of LNPs containing ApoE (151-299) and PD1, or a fusion protein containing ApoE (206-299) and PD1 was significantly increased (FIG. 6).

Based on these results, it was confirmed that the use of ApoE variants with partial deletions at the N-terminus leads to a significant enhancement in targeting efficiency.

### Example 3: Evaluation of Targeting Efficiency of Delivery Carrier Containing Fusion Proteins Including ApoE Variants and SIRPα.

In Example 1, the targeting efficacy of a fusion protein including an ApoE protein (or a variant thereof) and a targeting protein was confirmed, and PD1 was used as an example of the targeting protein. Accordingly, to confirm that targeting efficacy is enhanced by the ApoE protein even when various targeting proteins are employed, SIRPα (SEQ ID NO: 10) was used as another example of a targeting protein.

Meanwhile, SIRPα (Signal Regulatory Protein Alpha) and CD47 are known to play important roles in the immune system. Tumor cells are known to overexpress CD47, which may bind to SIRPα expressed on macrophages. Therefore, by using a fusion protein containing SIRPα, the targeting efficiency toward cells expressing CD47 was evaluated.

Specifically, the fusion protein containing an ApoE protein variant and SIRPα as a targeting protein was mixed with a luciferase-loaded lipid nanoparticle in the same manner as in Example 1 to produce a lipid nanoparticle (targeting carrier) to which the fusion protein was linked, and after treatment on A549 cells (control) or A549-CD47 cells (A549 expressing hCD47 on the surface), a luciferase assay was performed with cell lysates 24 hours later to evaluate the delivery efficiency of the targeting carrier. In addition, to evaluate the delivery efficiency of the fusion protein, lipid nanoparticles linked to ApoE (206-299) protein or SIRPα were prepared and used as controls.

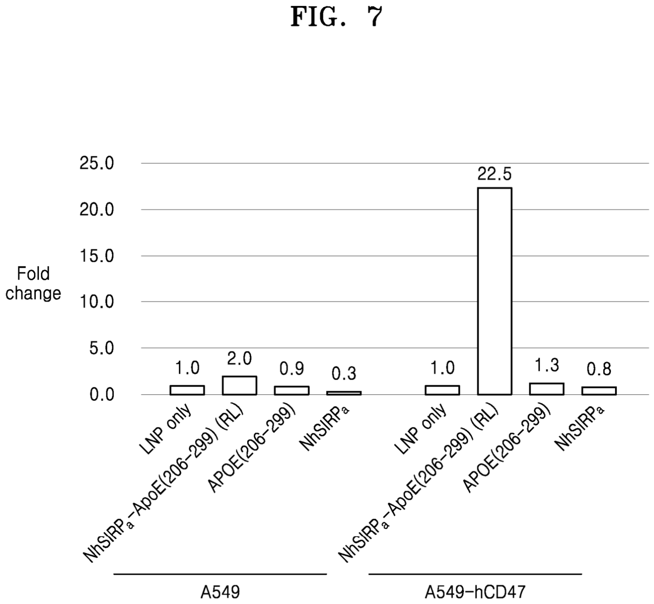

As a result, it was confirmed that the delivery efficiency of LNPs conjugated with the fusion protein including ApoE (206-299) and SIRPα was significantly increased compared to other comparative groups (FIG. 7).

Based on these results, it was confirmed that the use of ApoE variants with partial deletions at the N-terminus leads to a significant improvement in targeting efficiency, regardless of the type of targeting protein employed.

The description of the present disclosure is for illustrative purposes only, and a person having ordinary skill in the art to which the present disclosure pertains will understand that the present disclosure may be easily modified into other specific forms without changing the technical idea or essential characteristics of the present disclosure. Therefore, it should be understood that the embodiments described above are exemplary in all respects and not limiting.

## Claims

1. A fusion protein comprising:
1) an apolipoprotein or a fragment thereof; and
2) a targeting protein or a fragment thereof.

2. The fusion protein of claim 1, wherein the apolipoprotein comprises at least one selected from the group consisting of apolipoprotein A (ApoA protein), apolipoprotein B (ApoB protein), apolipoprotein C (ApoC protein), apolipoprotein D (ApoD protein), apolipoprotein E (ApoE protein), apolipoprotein F (ApoF protein), apolipoprotein H (ApoH protein), apolipoprotein L (ApoL protein), apolipoprotein M (ApoM protein), and apolipoprotein(a) (Apo(a) protein).

3. The fusion protein of claim 2, wherein the ApoE protein comprises at least one selected from the group consisting of ApoE1, ApoE2, ApoE3, ApoE4, ApoE5, ApoE6, ApoE7, ApoE8, ApoE9, ApoE10, and ApoE11.

4. The fusion protein of claim 1, wherein the fragment of the apolipoprotein is one in which a receptor-binding region is deleted.

5. The fusion protein of claim 1, wherein the fragment of the apolipoprotein is one in which both a receptor-binding region and a hinge region are deleted.

6. The fusion protein of claim 1, wherein the fragment of the apolipoprotein is one in which 100 to 150 amino acids from the N-terminus of the apolipoprotein are deleted.

7. The fusion protein of claim 1, wherein the fragment of the apolipoprotein is one in which 151 to 220 amino acids from the N-terminus of the apolipoprotein are deleted.

8. The fusion protein of claim 1, wherein the apolipoprotein comprises a peptide represented by the amino acid sequence of SEQ ID NO: 1 or SEQ ID NO: 2.

9. The fusion protein of claim 1, wherein the fragment of the apolipoprotein comprises a peptide represented by an amino acid sequence in which 100 to 220 amino acids from the N-terminus of SEQ ID NO: 2 are deleted.

10. The fusion protein of claim 1, wherein the fragment of the apolipoprotein comprises a peptide represented by the amino acid sequence of SEQ ID NO: 3 or SEQ ID NO: 4.

11. The fusion protein of claim 1, wherein the targeting protein comprises at least one selected from the group consisting of PD-1, CTLA-4, CD28, ICOS, OX40, OX40L, GITR, GITRL, CD40, CD40L, CD47, SIRP-alpha, 4-1BB, 4-1BBL, B7-1, B7-2, B7-H1, B7-H2, B7-DC, CD80, CD160, BTLA, HVEM, DPP-4, NTCP, CD16, VEGF, VEGFR, Caveolin-1, TMIGD2, LIGHT, LAG3, CD27, NAM-1, CD96, 2B4, TIM-3, Adenosine A2a receptor, CEACAM1, DC-SIGN, CD200R, DR3, HVEM (CD270), CD137, TIGIT and CD112R.

12. The fusion protein of claim 1, wherein the targeting protein comprises an antibody or an antigen-binding fragment thereof, capable of binding to one or more selected from the group consisting of PD-L1, PD-L2, MAGE-1, MAGE-2, MAGE-3, MAGE-12, BAGE, GAGE, NY-ESO-1, tyrosinase, TRP-1, TRP-2, gp100, MART-1, MCIR, Ig idotype, CDK4, Caspase-B, beta-catenin, CLA, BCR/ABL, mutated p21/ras, mutated p53, proteinase 3, WT1, MUC-1, Her2/neu, PAP, PSA, PSMA, G250, HPV E6/E7, EBV LMP2a, CEA, alpha-Fetoprotein, 5T4, CD47, and onco-trophoblast glycoprotein.

13. A polynucleotide comprising a sequence encoding the fusion protein of any one of claims 1 to 12.

14. An expression vector comprising the polynucleotide of claim 13.

15. A targeting carrier comprising the fusion protein of any one of claims 1 to 12 and a lipid-based carrier.

16. The targeting carrier of claim 15, wherein the targeting carrier targets a protein to which the targeting protein included in the fusion protein is capable of binding, or a cell expressing the protein.

17. The targeting carrier of claim 15, wherein the targeting carrier targets cancer.

18. A composition for drug delivery, comprising the targeting carrier of claim 15 and a drug of interest.

19. The composition of claim 18, wherein the composition is for delivering a drug to a target cell or tissue.

20. The composition of claim 18, wherein the composition is for delivering a drug to a cancer cell or a cancer lesion.

21. The composition according to claim 20, wherein the cancer is liver cancer, lung cancer, pancreatic cancer, non-small cell lung cancer, colon cancer, bone cancer, skin cancer, head or neck cancer, cutaneous or intraocular melanoma, uterine cancer, ovarian cancer, rectal cancer, gastric cancer, perianal cancer, colorectal cancer, breast cancer, fallopian tube carcinoma, endometrial carcinoma, cervical carcinoma, vaginal carcinoma, vulvar carcinoma, Hodgkin's disease, esophageal cancer, small intestinal cancer, endocrine gland cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureter cancer, renal cell carcinoma, renal pelvic carcinoma, central nervous system (CNS) tumor, primary CNS lymphoma, spinal tumor, brainstem glioma, or pituitary adenoma.
